# EUROPEAN PATENT SPECIFICATION

(11) **EP 2 493 293 B1**
(45) Date of publication and mention of the grant of the patent: **02.03.2016**
(21) Application number: 10830307.4
(22) Date of filing: 22.10.2010
(51) Int. Cl.: A61K 31/015, A61P 27/12, A61P 27/00, A61P 43/00

(54) **METHODS FOR MAINTAINING EYE HEALTH AND AMELIORATING OPTHALMIC MALADIES IN CANINES**
VERFAHREN ZUR GESUNDERHALTUNG DER AUGEN UND ZUR LINDERUNG VON AUGENLEIDEN BEI HUNDEN
PROCÉDÉS DE MAINTIEN DE LA SANTÉ OCULAIRE ET D'AMÉLIORATION DES MALADIES OPHTALMIQUES CHEZ LES CANIDÉS

(30) Priority: 30.10.2009 US 280175 P
(43) Date of publication of application: 05.09.2012
(73) Proprietor: Nestec S.A., 1800 Vevey (CH)
(72) Inventor: PAN, Yuanlong, Chesterfield MO 63017 (US); WANG, Wei, Olivette MO 63132 (US)
(74) Representative: Rupp, Christian
(86) International application number: PCT/US2010/002815
(87) International publication number: WO 2011/059474

(56) References cited:
- WO-A1-00/44375
- WO-A2-2006/116755
- RU-C1- 2 094 060
- US-A1- 2004 259 187
- US-A1- 2007 082 044
- US-A1- 2007 286 925
- US-A1- 2008 254 140
- US-A1- 2009 196 921
- YEUM K -J ET AL: "Measurement of carotenoids, retinoids, and tocopherols in human lenses", INVESTIGATIVE OPHTHALMOLOGY AND VISUAL SCIENCE 1995 US, vol. 36, no. 13, 1995, pages 2756-2761, ISSN: 0146-0404

## Description

### BACKGROUND OF THE INVENTION

### Field of the Invention

The invention relates generally to methods for maintaining eye health and ameliorating ophthalmic maladies and particularly to methods for using carotenes to maintain eye health and ameliorate ophthalmic maladies in canines.

### Description of Related Art

Ophthalmic maladies such as cataracts, retinal degeneration, and cloudy eye are common in canines, particularly canines that are outdoors and exposed to excessive sunlight and to aging canines. Methods for counteracting some such maladies are known in the art. WO2009051223 discloses using vascular-adhesion-protein-1 (VAP-1) inhibitors for treating cataracts. US20090082415 discloses using a mixture of topical carriers having carbomer, glycerin, ethylene diamine tetraacetic acid, benzalkonium chloride, balance water, and aldose reductase inhibitors for treating optical complications of diabetes in dogs. US7442711 discloses using N-hydroxy piperidine compounds for the amelioration of cataracts and other ophthalmic diseases. US20060084685 discloses using roflumilast for the treatment of various eye diseases. These methods are, however, insufficient to solve all the problems associated with these and other ophthalmic maladies. There is, therefore, a need for new methods for ameliorating ophthalmic maladies in canines.

RU 2 094 060 relates to the use of compositions comprising unspecified carotenoids in an ointment, that means the compositions are applied topically to the eye.

The difference between the subject matter of the independent claims and RU 2 094 060 is inter alia that the treatment group are canines, the carotene is specified to be alpha-carotene and that the compositions are administered orally.

### SUMMARY OF THE INVENTION

The invention is directed to a composition comprising α-carotene in amounts such that α-carotene is accumulated in the eye tissue for use in preventing or ameliorating ophthalmic maladies in canines, wherein the malady is cloudy eye and the composition is to be administered orally.

In an embodiment of the invention the α-carotene has accumulated in the cortical eye tissue, the nuclear eye tissue, or both.

In a further embodiment of the invention α-carotene is administered to the canines in amounts of from about 0.1 to about 5000 mg; optionally wherein α-carotene is administered to the canines in amounts of from about 0.1 to about 500 mg per day.

In a further embodiment of the invention the canines are (i) dogs or (ii) aging canines.

In a further embodiment of the invention α-carotene is administered to the canines on an extended regular basis; optionally wherein α-carotene is administered to the canines on a daily basis.

In a further embodiment of the invention the composition is for co-administration in conjunction with one or more ophthalmic agents in an amount effective for preventing or ameliorating cloudy eye.

The invention is also directed to the use of α-carotene to prepare a medicament for preventing or ameliorating cloudy eye in canines and the composition is to be administered orally.

Other and further objects, features, and advantages of the present invention will be readily apparent to those skilled in the art.

The scope of the invention is limited by the appended claims.

### DETAILED DESCRIPTION

### Definitions

The term "radiation" means electromagnetic waves having wavelengths that are partially or completely absorbed by carotenes. Generally, carotenes such as β-carotene absorb electromagnetic waves having wavelengths from about 350 to about 550 nanometers (nm), with the best absorption occurring at from about 400 to about 500 nm.

The term "ophthalmic agent" means any compound, composition, or drug, other than carotenes, useful for ameliorating ophthalmic maladies in canines.

The term "in conjunction" means that one or more carotenes and one or more ophthalmic agents are administered to canines (1) together, e.g., in a food composition, or (2) separately at the same or different frequency using the same or different administration routes at about the same time or periodically. "Periodically" means that the agent is administered on a dosage schedule acceptable for a specific agent and that the food is fed to canines routinely as appropriate for the particular animal. "About the same time" generally means that the food and agent are administered at the same time or within about 72 hours of each other. "In conjunction" specifically includes administration schemes wherein an ophthalmic agent is administered for a prescribed period and carotenes are administered indefinitely.

The term "single package" means that the components of a kit are physically associated, in or with one or more containers, and considered a unit for manufacture, distribution, sale, or use. Containers includebags, boxes or cartons, bottles, packages of any type or design or material, over-wrap, shrink-wrap, affixed components (e.g., stapled, or adhered), or combinations of any of the foregoing. For example, a single package kit may provide containers of individual compounds and/or compositions physically associated such that they are considered a unit for manufacture, distribution, sale, or use.

The term "virtual package" means that the components of a kit are associated by directions on one or more physical or virtual kit components instructing the user how to obtain the other components, e.g., in a bag or other container containing one component and directions instructing the user to go to a website, contact a recorded message or a fax-back service, view a visual message, or contact a caregiver or instructor to obtain, for example, instructions on how to use the kit, or safety or technical information about one or more components of a kit. Examples of information that can be provided as part of a virtual kit include instructions for use; safety information such as material safety data sheets; poison control information; information on potential adverse reactions; clinical study results; dietary information such as food composition or caloric composition; general information on ophthalmic maladies; diseases that affect ophthalmic function, or general information on treatment or preservation of ophthalmic maladies; self-help relating to ophthalmic maladies; caregiver information for those caring for canines with ophthalmic maladies; and use, benefits, and potential side-effects or counter-indications for ophthalmic agents.

The term "health and wellness of an animal" means the complete physical, mental, and social well being of the animal, not merely the absence of disease or infirmity.

The term "quality of life" means the ability to enjoy normal life activities.

The term "extending the prime" means extending the number of years an animal lives a healthy life and not just extending the number of years an animal lives, e.g., an animal would be healthy in the prime of its life for a relatively longer time.

As used throughout, ranges are used herein in shorthand, so as to avoid having to set out at length and describe each and every value within the range. Any appropriate value within the range can be selected, where appropriate, as the upper value, lower value, or the terminus of the range.

As used herein and in the appended claims, the singular form of a word includes the plural, and vice versa, unless the context clearly dictates otherwise. Thus, the references "a", "an", and "the" are generally inclusive of the plurals of the respective terms. For example, reference to "a canine", "a method", or "an agent" includes a plurality of such "canines", "methods", or "agents". Similarly, the words "comprise", "comprises", and "comprising" are to be interpreted inclusively rather than exclusively. Likewise the terms "include", "including" and "or" should all be construed to be inclusive, unless such a construction is clearly prohibited from the context.

Unless defined otherwise, all technical and scientific terms, terms of art, and acronyms used herein have the meanings commonly understood by one of ordinary skill in the art in the field(s) of the invention, or in the field(s) where the term is used. Although any compositions, methods, articles of manufacture, or other means or materials similar or equivalent to those described herein can be used in the practice of the present invention, the preferred compositions, methods, articles of manufacture, or other means or materials are described herein.

The discussion of those references is intended merely to summarize the assertions made therein. No admission is made that any such patents, patent applications, publications or references, or any portion thereof, are relevant, material, or prior art. The right to challenge the accuracy and pertinence of any assertion of such patents, patent applications, publications, and other references as relevant, material, or prior art is specifically reserved. Full citations for publications not cited fully within the specification are set forth at the end of the specification.

### The Invention

In one aspect, the invention is directed to a composition comprising α-carotene in amounts such that α-carotene is accumulated in the eye tissue for use in preventing or ameliorating ophthalmic maladies in canines, wherein the malady is cloudy eye and the composition is to be administered orally.. The invention is based upon the unexpected discovery that carotenes accumulate in canine eye tissue and the knowledge that carotenes are effective for absorbing radiation that damages the eye and for ameliorating damage caused to the eye by free radicals. The discovery contradicts current beliefs that carotenes are not accumulated in canine eye tissue and the knowledge that carotenes do not accumulate in human eye tissue. As disclosed by Yeum (Yeum KJ, Taylor A, Tang G, Russell RM. Measurement of carotenoids, retinoids, and tocopherols in human lenses. Invest Ophthalmol.Vis.Sci 1995;36:2756-61), eye tissue is known to contain xanthophylls such as lutein and zeaxanthin but is known not to contain carotenes such as β-carotene and lycopene.

Since eye tissue does not synthesize carotenes, any carotenes in the eye must be derived from external sources, e.g., the diet. For known eye tissue, i.e., human eye tissue, the consumption of carotenes produces a short term systemic elevation of carotenes. In contrast, canine eye tissue selectively absorbs carotenes and keeps carotenes within the eye tissue, i.e., canine eye tissue accumulates carotenes. These carotenes remain in the eye even when carotenes are not being consumed regularly and the systemic level of carotenes has declined. This accumulation of carotenes provides protection against radiation and free radicals and ameliorates ophthalmic maladies in the canines caused by radiation and free radicals. Therefore, at least for canines, the surprising discovery that carotenes accumulate in canine eye tissue gives the canine an advantage over other animals because the accumulated carotenes absorb harmful electromagnetic radiation and scavenges free radicals that are known to cause ophthalmic maladies.

The carotenes accumulate in the eyes, including eye tissue susceptible to damage from radiation and free radicals. The carotenes accumulate in the cortical eye tissue, the nuclear eye tissue, or both. Carotenes that accumulate in such tissue provide protection to the lens and related tissue by preventing damage by radiation and free radicals.

The ophthalmic malady ameliorated by the invention is cloudy eye

The carotenes useful in the invention are any carotenes suitable for administration to canines. Such carotenes and their sources are known to skilled artisans. Generally, carotenes are obtained from any suitable source, synthetic, natural, and combinations thereof. A preferred source of carotenes is dietary carotenes contained in many fruits and vegetables, e.g., carrots, pumpkins, sweet potatoes, spinach, Vietnamese gac, palm oil, mangoes, broccoli, palayas yams, kale, beet greens, winter squash, chard, and the like. Other sources include synthetic carotenes, e.g., as dietary supplements in tablet, capsule, and similar forms.

The carotene for use in the invention is α-carotene.

The carotenes are to be administered to the canines in any amount that is effective for ameliorating ophthalmic maladies. In various embodiments, the carotenes are administered to the canine in amounts of from about 0.1 to about 5000 mg as desired or needed, preferably from about 0.5 to about 3000 mg, most preferably from about 1 to about 1000 mg. In other embodiments, the carotenes are administered in amounts of from about 0.1 to about 500 mg per day, preferably from about 0.5 to about 250 mg per day, most preferably from about 1 to about 100 mg per day. The amount may vary within the range depending on the malady and the characteristics of the canine.

The carotenes are administered to the canines orally. In various embodiments, administration is on an as-needed or as-desired basis of varying or regular frequency depending on the intended use. In other embodiments, administration is on a regular or scheduled basis. Administration on a regular or scheduled basis provides the canines with a regular and consistent dose of carotenes that keeps the level of carotenes in the eye relatively constant. Regular or scheduled administration can be once monthly, once weekly, once daily, or more than once daily. Similarly, administration can be every other day, week, or month, every third day, week, or month, every fourth day, week, or month, and the like. Administration can be multiple times per day. Such administration can depend on the age, size, health, malady, and the like for the canine. In some embodiments, carotenes or comestible ingredients containing carotenes are applied to or admixed with typical foods and drinks and administered to the canines, e.g., carotenes are an integral part of a canine pet food. In one embodiment, administration is part of a dietary regimen. For example, such a dietary regimen comprises the regular ingestion of one or more carotenes or any comestible ingredient containing such carotenes in an amount effective for ameliorating ophthalmic maladies. In preferred embodiments, the carotenes are administered to the canines as part of a food composition or in the form of a dietary supplement. Preferably, the carotenes are administered to the canines as part of a wet or dry pet food composition, e.g., kibbles or wet canned pet food. In a preferred embodiment, the carotenes are administered on an extended regular basis, preferably a daily basis.

In various embodiments, the canines are wolves, foxes, jackals, coyotes, and dogs, preferably dogs, most preferably domestic dogs.

Carotene administration, including administration as part of a dietary regimen, can span a period ranging from parturition through the life of the canine. In certain embodiments, the canine is a young, growing, or adult canine. In preferred embodiments, the canine is an aging canine susceptible to or suffering from ophthalmic maladies characteristic of aging. In other embodiments, the canine is a canine that is likely to be exposed to excess radiation that will adversely impact the eye, e.g., a hunting dog or a working dog.

In one embodiment, α-carotene is to be administered in conjunction with one or more ophthalmic agents in an amount effective for ameliorating ophthalmic maladies as defined herein. In a particular embodiment, α-carotene is to be administered in a pharmaceutical composition that includes one or more ophthalmic agents along with one or more further carotenes. In a preferred embodiment, the carotenes are to be administered to the animal on a daily basis, preferably as part of a food composition or in a dietary supplement, and the ophthalmic agents are to be administered on an as prescribed basis, generally in the form of a pharmaceutical composition such as a pill. Such agents include avastin (e.g., for radiation retinopathy); antibiotics; anti-inflammatory compounds (e.g., COX-2 inhibitors); tranquilizers; antioxidants such as resveratrol, proanthocyanidins, anthocyanins, carotenoids (e.g., lutein, zeaxanthin, astaxanthin, cryptoxanthin, and lycopene); bioflavonoids (e.g., taxifolin, naringenin, and hesperetin); glutathione; catechin; epicatechin; epigallocatechin; epigallocatechin gallate; epicatechin gallate; and lipoic acid; and vitamins, e.g., vitamin A, vitamin E, and vitamin C. Skilled artisans can determine the proper type and amount of ophthalmic agents to administer to the canines based upon their age, size, health, malady, and the like for the canine.

Compositions of the invention for use in ameliorating ophthalmic maladies have many beneficial effects for canines. Many day-to-day life functions are dependent upon or related to ophthalmic function. Eliminating or lessening the impact and severity of ophthalmic maladies that interfere with normal ophthalmic function, particularly those that impair vision, improve many aspects of life. For example, ameliorating ophthalmic maladies is related to (1) maintaining eye health; (2) maintaining or improving vision; (3) preventing or ameliorating cataracts; (4) preventing or ameliorating cloudy eye; (5) preventing or ameliorating canine eye injury caused by radiation; (6) preventing or ameliorating retinal degeneration; (7) reducing or preventing a decline of social interaction; (8) promoting overall health and wellness; (9) improving the quality of life; and (10) extending the prime. Therefore, in various aspects, the invention provides compositions for affecting various ophthalmic related functions adversely affected by these ophthalmic maladies. In all these aspects, the benefit results from an accumulation of carotenes in the eyes of the canine, i.e., in the eye tissue exposed to radiation and susceptible to free radical damage.

Not covered by the invention but disclosed herein are the following aspects:
In one such aspect, methods are disclosed for maintaining eye health in canines comprising administering to the canines an eye health maintaining amount of one or more carotenes. The carotenes maintain overall eye health by preventing ophthalmic maladies in canines, particularly those caused by radiation and by free radicals.

In another such aspect, methods are disclosed for maintaining or improving vision in canines comprising administering to the canines a vision maintaining or improving amount of one or more carotenes. The carotenes absorb radiation and scavenge free radicals that are at least partially responsible for causing ophthalmic maladies that impair vision. With no or less severe ophthalmic maladies, the canines have better vision.

In another aspect, methods are disclosed for preventing or ameliorating cataracts in canines comprising administering to the canines a cataract preventing or ameliorating amount of one or more carotenes. The carotenes absorb radiation and scavenge free radicals that are at least partially responsible for causing cataracts in canines. With no or less severe cataracts, the canines have better vision and overall eye health.

In another aspect, methods are disclosed for preventing or ameliorating cloudy eye in canines comprising administering to the canines a cloudy eye preventing or ameliorating amount of one or more carotenes. The carotenes absorb radiation and scavenge free radicals that are at least partially responsible for causing cloudy eye in canines. With no or less severe cloudy eye, the canines have better vision and have a more healthy and appealing appearance.

In a further aspect, methods are disclosed for preventing or ameliorating canine eye injury caused by radiation comprising administering to the canines an eye injury preventing or ameliorating amount of one or more carotenes. The carotenes absorb radiation that damages eye tissue and causes injury to the eye.

In another aspect, methods are disclosed for preventing or ameliorating retinal degeneration in canines comprising administering to the canines a retinal degeneration preventing or ameliorating amount of one or more carotenes. The carotenes absorb radiation and scavenge free radicals that are at least partially responsible for causing retinal degeneration in canines. With no or less severe retinal degeneration, the canines have better vision and have a more healthy and appealing appearance.

In yet another aspect, methods are disclosed for reducing or preventing a decline of social interaction in canines comprising administering to the canines a social interaction decline reducing or preventing amount of one or more carotenes. The carotenes absorb radiation and scavenge free radicals that are at least partially responsible for impairing vision in canines. With better vision, the canines are more socially interactive, particularly when participating in activities that require good vision, e.g., fetch. The methods ensure that the canines remain involved in playtime, participate in group activities, interact with caregivers, and the like.

In one aspect, methods are disclosed for promoting the health and wellness of canines comprising administering to the canines a health and wellness promoting amount of one or more carotenes. The carotenes improve eye health and vision and improve the canine's overall health and wellness.

In another aspect, methods are disclosed for improving the quality of life for canines comprising administering to the canines a quality of life improving amount of one or more carotenes. The carotenes improve eye health and vision and contribute to the quality of life of the canines.

In a further aspect, methods are disclosed for extending the prime for canines comprising administering to the canines a prime extending amount of one or more carotenes. The methods extend the time the canines have good vision and delay the onset of ophthalmic maladies so that the canines have a longer period during their life wherein they have good vision and related ophthalmic functions.

In the methods for maintaining eye health, maintaining or improving vision, preventing or ameliorating cataracts, preventing or ameliorating cloudy eye, preventing or ameliorating retinal degeneration, preventing or ameliorating canine eye injury caused by radiation, reducing or preventing a decline of social interaction, promoting overall health and wellness, improving the quality of life, and extending the prime, the amount of carotenes used in these methods are the same as the amounts or within the ranges given herein for ameliorating ophthalmic maladies. Administration routes, specific compounds, and other parameters are the same as described herein for ameliorating ophthalmic maladies.

In a further aspect, kits are disclosed suitable for administering carotenes to canines to ameliorate ophthalmic maladies. The kits comprise in separate containers in a single package or in separate containers in a virtual package, as appropriate for the kit component, instructions for how to use carotenes to ameliorate ophthalmic maladies and at least one of (1) one or more carotenes; (2) one or more comestible ingredients containing carotenes; (3) one or more comestible ingredients compatible with carotenes; (4) one or more ophthalmic agents; (5) instructions for how to combine or prepare the carotenes and any other ingredients provided in the kit for administration to canines; and (6) a device for administering the combined or prepared kit components to canines. The components are each provided in separate containers in a single package or in mixtures of various components in different packages. In preferred embodiments, the kits comprise the instructions and one or more carotenes or comestible ingredients containing carotenes. The kits may comprise the ingredients in various combinations.

In another aspect, a means are disclosed for communicating information about or instructions for one or more of (1) using carotenes for ameliorating ophthalmic maladies; (2) using carotenes in conjunction with ophthalmic agents; (3) admixing carotenes with other materials to produce a composition suitable for ameliorating ophthalmic maladies; (4) using the kits for ameliorating ophthalmic maladies; (5) benefits of using carotenes for ameliorating ophthalmic maladies; and (6) administering carotenes to canines. The means comprises one or more of a physical or electronic document, digital storage media, optical storage media, audio presentation, audiovisual display, or visual display containing the information or instructions. Preferably, the means is selected from the group consisting of a displayed website, a visual display kiosk, a brochure, a product label, a package insert, an advertisement, a handout, a public announcement, an audiotape, a videotape, a DVD, a CD-ROM, a computer readable chip, a computer readable card, a computer readable disk, a USB device, a FireWire device, a computer memory, and any combination thereof.

In another aspect, packages are disclosed comprising one or more carotenes and a label affixed to the packages containing a word or words, picture, design, acronym, slogan, phrase, or other device, or combination thereof, that indicates that the contents of the package contains carotenes that are suitable for one or more of ameliorating ophthalmic maladies; maintaining eye health; maintaining or improving vision; preventing or ameliorating cataracts; preventing or ameliorating cloudy eye; preventing or ameliorating retinal degeneration; preventing or ameliorating canine eye injury caused by radiation; reducing or preventing a decline of social interaction; promoting overall health and wellness; improving the quality of life; and extending the prime for canines. Typically, such device comprises the words 'prevents damage caused by ultraviolet light, "maintains eye health, "prevents eye damage caused by radiation", "helps prevent cataracts", "helps prevent cloudy eye in dogs", or an equivalent expression printed on the package. Any package or packaging material suitable for containing the carotenes is useful, e.g., a bag, box, bottle, can, and pouch, manufactured from paper, plastic, foil, and metal. The package can contain a food composition adapted for a canine that supplies the required amount of carotenes to the canine, e.g., a pet food composition formulated for canines.

In another aspect, a use of one or more carotenes to prepare a medicament for one or more of ameliorating ophthalmic maladies is disclosed; maintaining eye health; maintaining or improving vision; preventing or ameliorating cataracts; preventing or ameliorating cloudy eye; preventing or ameliorating retinal degeneration; preventing or ameliorating canine eye injury caused by radiation; reducing or preventing a decline of social interaction; promoting overall health and wellness; improving the quality of life; and extending the prime for canines. Generally, medicaments are prepared by admixing carotenes with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to canines.

### EXAMPLES

### Reference Example 1

Eye samples were collected from seven (7) dogs. Eye balls were removed within 1 hour of death at necropsy, stored in individual containers at 4°C, and stored on wet ice for dissection within one day. The eyes were dissected to take out lenses. The lenses were separated into two layers: (1) an outer, soft cortical/epithelial layer and (2) an inner, hard nuclear layer. The layers were analyzed for carotenoids, vitamin C, and vitamin E following standard procedures given in Craft NE, Haitema TB, Garnett KM, Fitch KA, Dorey CK. Carotenoid, tocopherol, and retinol concentrations in elderly human brain. J Nutr. Health Aging 2004;8:156-62. The results are given in Table 1.

**Table 1**

| Antioxidant Levels in Different Layers of Dog Lens | | |
|---|---|---|
| *Variables* | *Cortical*/*Epithelial Layer (C)* | *Nuclear Layer (N)* |
| β-carotene (ng/g) | 1.74 | 2.74 |
| Vitamin C (µg/g) | 112.38 | 23.59 |
| Vitamin E (µg/g) | 0.93 | 0.64 |

| | | |
|---|---|---|
| Nanograms (ng); grams (g). | | |

Referring to Table 1, the data show that all nuclear layer samples accumulated β-carotene and that six out of seven cortical layer samples accumulated β-carotene.

**Table 2**

| Concentrations of β-Carotene in Different Layers of Individual Dog Lens | | | | | | |
|---|---|---|---|---|---|---|
| Dog # | Age | Breed | Sex | Lens Layers | Lens Weight (grams) | β-Carotene (ng/g) |
| 1 | 15.50 | BEA | M/n | C | 0.23 | nd* |
| | | | | N | 0.38 | 0.79 |
| 2 | 13.55 | BEA | M/n | C | 0.22 | 1.06 |
| | | | | N | 0.32 | 0.60 |
| 3 | 13.41 | LAB | F/s | C | 0.57 | 0.73 |
| | | | | N | 0.27 | 1.26 |
| 4 | 14.22 | LAB | M/n | C | 0.45 | 0.71 |
| | | | | N | 0.23 | 0.79 |
| 5 | 12.79 | Man T | F/s | C | 0.37 | 1.35 |
| | | | | N | 0.19 | 1.60 |
| 6 | 6.10 | SIB HUSKY | M/n | C | 0.25 | 4.26 |
| | | | | N | 0.26 | 8.17 |
| 7 | 9.31 | LAB | F/s | C | 0.37 | 2.35 |
| | | | | N | 0.26 | 5.99 |

| | | | | | | |
|---|---|---|---|---|---|---|
| * Detection limit for carotenoids is 0.2 ng/g. * Neutered (n); Spayed (s). | | | | | | |

Referring to Table 2, the data show that different types and sexes of dogs accumulate β-carotene in different types of eye tissue. In contrast, previous studies (See, Bates CJ, Chen SJ, Macdonald A, Holden R. Quantization of vitamin E and a carotenoid pigment in cataractous human lenses, and the effect of a dietary supplement. Int.J Vitam. Nutr. Res 1996:66:316-21; Bernstein PS, Khachik F, Carvalho LS, Muir GJ, Zhao DY, Katz NB. Identification and quantization of carotenoids and their metabolites in the tissues of the human eye. Exp. Eye Res 2001:72:215-23; Yeum KJ, Taylor A, Tang G, Russell RM. Measurement of carotenoids, retinoids, and tocopherols in human lenses. Invest Ophthalmol. Vis. Sci 1995:36:2756-61; and Yeum KJ, Shang FM, Schalch WM, Russell RM, Taylor A. Fat-soluble nutrient concentrations in different layers of human cataractous lens. Curr. Eye Res 1999;19:502-5) showed that β-carotene was not detected or accumulated in human lenses even though humans regularly consume β-carotene in their diets. These data in Table 2 show that canine eye tissue is very unique because it accumulates β-carotene. β-Carotene as a yellow-pigment blocks electromagnetic radiation that damages the eyes. In addition, β-carotene is a powerful antioxidant that prevents and reduces free radicals formation in the eyes. Therefore, β-carotene plays a unique and critical role in maintaining eye health and ameliorating ophthalmic maladies in canines such as dogs.

In the specification, there have been disclosed typical preferred embodiments of the invention. Although specific terms are employed, they are used in a generic and descriptive sense only and not for purposes of limitation. The scope of the invention is set forth in the claims. Obviously many modifications and variations of the invention are possible in light of the above teachings. It is therefore to be understood that within the scope of the appended claims the invention may be practiced otherwise than as specifically described.

## Claims

1. A composition comprising α-carotene in amounts such that α-carotene is accumulated in the eye tissue for use in preventing or ameliorating ophthalmic maladies in canines, wherein the malady is cloudy eye and the composition is to be administered orally.

2. The composition for use according to claim 1 wherein the α-carotene has accumulated in the cortical eye tissue, the nuclear eye tissue, or both.

3. The composition for use according to claim 1 wherein α-carotene is administered to the canines in amounts of from about 0.1 to about 5000 mg; optionally wherein α-carotene is administered to the canines in amounts of from about 0.1 to about 500 mg per day.

4. The composition for use according to claim 1 wherein the canines are (i) dogs or (ii) aging canines.

5. The composition for use according to claim 1 wherein α-carotene is administered to the canines on an extended regular basis; optionally wherein α-carotene is administered to the canines on a daily basis.

6. The composition for use according to claim 1 wherein said composition is for co-administration in conjunction with one or more ophthalmic agents in an amount effective for preventing or ameliorating cloudy eye.

7. Use of α-carotene to prepare a composition for preventing or ameliorating cloudy eye in canines wherein the composition is to be administered orally.

## Patentansprüche

1. Zusammensetzung, die α-Carotin in Mengen umfasst, durch die α-Carotin im Augengewebe angesammelt wird, zur Verwendung bei der Prävention oder Linderung von Augenerkrankungen bei Hunden, wobei die Erkrankung trübe Augen sind und die Zusammensetzung oral zu verabreichen ist.

2. Zusammensetzung zur Verwendung nach Anspruch 1, wobei sich das α-Carotin im kortikalen Augengewebe, im Augenkerngewebe oder beidem angesammelt hat.

3. Zusammensetzung zur Verwendung nach Anspruch 1, wobei den Hunden α-Carotin in Mengen von etwa 0,1 bis etwa 5000 mg verabreicht wird; wobei den Hunden optional α-Carotin in Mengen von etwa 0,1 bis etwa 500 mg pro Tag verabreicht wird.

4. Zusammensetzung zur Verwendung nach Anspruch 1, wobei die Hunde (i) Hunde oder (ii) alternde Hunde sind.

5. Zusammensetzung zur Verwendung nach Anspruch 1, wobei den Hunden α-Carotin auf längerer regelmäßiger Basis verabreicht wird; wobei den Hunden optional täglich α-Carotin verabreicht wird.

6. Zusammensetzung zur Verwendung nach Anspruch 1, wobei die Zusammensetzung zur gleichzeitigen Verabreichung zusammen mit einem oder mehreren Ophthalmika in einer Menge verabreicht wird, die wirksam ist, um trübe Augen zu verhindern oder zu lindern.

7. Verwendung von α-Carotin zur Herstellung einer Zusammensetzung zur Prävention oder Linderung von trüben Augen bei Hunden, wobei die Zusammensetzung täglich oral zu verabreichen ist.

## Revendications

1. Composition comprenant de l'α-carotène en des quantités telles que l'α-carotène s'accumule dans le tissu oculaire pour une utilisation dans la prévention ou l'amélioration de maladies ophtalmiques chez les canidés, où la maladie est l'oeil trouble et la composition est destinée à être administrée par voie orale.

2. Composition utilisable selon la revendication 1, dans laquelle l'α-carotène s'est accumulé dans le tissu oculaire cortical, le tissu oculaire nucléaire, ou les deux.

3. Composition utilisable selon la revendication 1, dans laquelle l'α-carotène est administré aux canidés en des quantités allant d'environ 0,1 à environ 5000 mg ; facultativement dans laquelle l'α-carotène est administré à des canidés en des quantités allant d'environ 0,1 à environ 500 mg par jour.

4. Composition utilisable selon la revendication 1, dans laquelle les canidés sont (i) des chiens ou (ii) des canidés vieillissants.

5. Composition utilisable selon la revendication 1, dans laquelle l'α-carotène est administré aux canidés sur une base régulière prolongée ; facultativement dans laquelle l'α-carotène est administré aux canidés sur une base quotidienne.

6. Composition utilisable selon la revendication 1, dans laquelle ladite composition est destinée à une co-administration en combinaison avec un ou plusieurs agents ophtalmiques en une quantité efficace pour prévenir ou améliorer l'oeil trouble.

7. Utilisation d'α-carotène pour préparer une composition pour la prévention ou l'amélioration de l'oeil trouble chez les canidés, où la composition est destinée à être administrée par voie orale.
